(19) Europäisches Patentamt
European Patent Office
Office européen des brevets

(11) **EP 4 403 161 A1**

(12) **EUROPEAN PATENT APPLICATION**
published in accordance with Art. 153(4) EPC

(43) Date of publication:
**24.07.2024 Bulletin 2024/30**

(21) Application number: **22869745.4**

(22) Date of filing: **18.08.2022**

(51) International Patent Classification (IPC):
*A61K 6/84* (2020.01)    *A61K 6/16* (2020.01)
*A61K 6/25* (2020.01)

(52) Cooperative Patent Classification (CPC):
**A61K 6/16; A61K 6/25; A61K 6/84**

(86) International application number:
**PCT/JP2022/031237**

(87) International publication number:
**WO 2023/042598 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.09.2021 JP 2021149210**

(71) Applicant: **Tokuyama Dental Corporation
Tokyo 110-0016 (JP)**

(72) Inventors:
• **KIRA, Ryuta
Tokyo 110-0016 (JP)**
• **MIYAKE, Hideaki
Tokyo 110-0016 (JP)**
• **AKIZUMI, Hironobu
Tokyo 110-0016 (JP)**

(74) Representative: **Gill Jennings & Every LLP
The Broadgate Tower
20 Primrose Street
London EC2A 2ES (GB)**

(54) **X-RAY OPAQUE FILLER MATERIAL, X-RAY OPAQUE DENTAL FILLER MATERIAL, METHOD FOR PRODUCING X-RAY OPAQUE FILLER MATERIAL, AND CURABLE DENTAL COMPOSITION**

(57)    Provided are an X-ray opaque filler that can impart X-ray opacity required for a dental curable composition, and besides, hardly reduces the transparency of a cured body and thus enables aesthetic restoration, a method of producing the X-ray opaque filler, and a dental curable composition using the X-ray opaque filler. Specifically, provided are an X-ray opaque filler, which is blended in a curable composition including a polymerizable monomer to impart X-ray opacity to the curable composition and a cured body thereof, the X-ray opaque filler including any powder selected from the group consisting of: first powder including crystalline rare earth metal fluoride particles as a main component and having a full width at half maximum of a maximum intensity peak derived from the crystalline rare earth metal fluoride particles of 0.3° or more in an X-ray diffraction pattern; and second powder obtained by subjecting the first powder to surface treatment, a method of producing the X-ray opaque filler, and a dental curable composition using the X-ray opaque filler.

EP 4 403 161 A1

## Description

Technical Field

The present invention relates to an X-ray opaque filler, a dental X-ray opaque filler, a method of producing the X-ray opaque filler, and a dental curable composition.

Background Art

In dental treatment, a cavity after removal of dental caries is filled with a dental filling material, and the cavity is then closed with a cured body by curing the dental filling material. In general, a curable composition including a polymerizable monomer, a filler, and a polymerization initiator as main components is used as such dental filling material.

In general, an inorganic oxide filler, particularly a silica-based filler is used as the filler to be blended in the curable composition. However, the silica-based filler has low X-ray opacity. Accordingly, at the time of radiography or computed tomography in the dental treatment, the cured body in the cavity is not imaged, and it becomes difficult to determine a treatment site.

As means for improving the X-ray opacity of a dental curable composition, there has been known a method including using a filler containing an atom having a large atomic number. For example, in Patent Literature 1, there is a description of "a radiopaque dental restoration material based on a polymerizable organic binder and a radiopaque component, and optionally an inorganic filler, the radiopaque dental restoration material containing, as the radiopaque component, a fluoride of a rare earth metal (atomic number: 57 to 71) of the periodic system of elements or a mixture of these fluorides in an amount of from 1 wt% to 50 wt% based on a total weight."

Citation List

Patent Literature

[PTL 1] JP 03-17803 B2

Summary of Invention

Technical Problem

However, when an X-ray opaque filler formed of a rare earth metal fluoride is used, the transparency of a cured body obtained by curing a dental curable composition is reduced along with an increase in blending amount of the filler, as shown in FIG. 2 of Patent Literature 1. Accordingly, when sufficient X-ray opacity is to be obtained, it becomes difficult to achieve aesthetic restoration.

In view of the foregoing, an object of the present invention is to provide an X-ray opaque filler that can impart X-ray opacity required for a dental curable composition, and besides, hardly reduces the transparency of a cured body and thus enables aesthetic restoration. In addition, another object of the present invention is to provide a dental X-ray opaque filler including the X-ray opaque filler, a method of producing the X-ray opaque filler, and a dental curable composition using the X-ray opaque filler. In the following description, the simple description "cured body" means a cured body of a curable composition or a dental curable composition, and a cured body obtained by curing a polymerizable monomer is referred to as "cured body of a polymerizable monomer."

Solution to Problem

In order to solve the above-mentioned problem, according to a first aspect of the present invention, there is provided an X-ray opaque filler, which is blended in a curable composition including a polymerizable monomer to impart X-ray opacity to the curable composition and a cured body thereof, the X-ray opaque filling including any powder selected from the group consisting of: first powder including crystalline rare earth metal fluoride particles as a main component and having a full width at half maximum of a maximum intensity peak (hereinafter also referred to as "maximum peak half width") derived from the crystalline rare earth metal fluoride particles of 0.3° or more in an X-ray diffraction pattern; and second powder obtained by subjecting the first powder to surface treatment.

In the X-ray opaque filler according to the above-mentioned aspect (hereinafter also referred to as "X-ray opaque filler of the present invention"), it is preferred that the first powder be powder including, as a main component, at least one kind of particles selected from the group consisting of: crystalline rare earth metal fluoride particles having an average primary particle diameter of from 5 nm to 300 nm measured with an electron microscope; and aggregated particles of the crystalline rare earth metal fluoride particles having an average primary particle diameter of from 1 nm to 500 nm. In addition, it is preferred that the crystalline rare earth metal fluoride particles be crystalline ytterbium fluoride particles. It is preferred that the crystalline rare earth metal fluoride particles include at least one kind of particles selected from the group consisting of: crystalline lanthanum fluoride particles; crystalline cerium fluoride particles; and crystalline gadolinium fluoride particles. It is preferred that the full width at half maximum be 0.77° or less. It is preferred that the full width at half maximum be from 0.47° to 0.68°. It is preferred that the full width at half maximum be from 0.51° to 0.59°.

According to a second aspect of the present invention, there is provided a dental X-ray opaque filler (hereinafter also referred to as "dental X-ray opaque filler of the present invention") including the X-ray opaque filler of the present invention.

According to a third aspect of the present invention, there is provided a method of producing the X-ray opaque filler of the present invention, including a step of subjecting raw material powder including crystalline rare earth metal fluoride particles as a main component and having a maximum peak half width derived from the crystalline rare earth metal fluoride particles of less than 0.3° to mechanochemical treatment so that the full width at half maximum becomes 0.3° or more.

In the production method according to the above-mentioned aspect (hereinafter also referred to as "production method of the present invention"), it is preferred that the mechanochemical treatment be wet bead mill treatment.

According to a fourth aspect of the present invention, there is provided a dental curable composition, including: a polymerizable monomer; and the X-ray opaque filler of the present invention.

In the dental curable composition according the above-mentioned aspect (hereinafter also referred to as "dental curable composition of the present invention"), it is preferred that the crystalline rare earth metal fluoride particles be crystalline ytterbium fluoride particles, and a cured body of the polymerizable monomer have a refractive index for a sodium d line at 25°C of from 1.45 to 1.60. In addition, it is preferred that the dental curable composition satisfy the following expression (1):

·

$$\cdot \text{Expression (1)} \quad -0.02 \leq (n_X - n_M) \leq 0.1$$

in the expression (1), $n_X$ represents a refractive index of the crystalline rare earth metal fluoride particles, and $n_M$ represents a refractive index of a cured body of the polymerizable monomer.

Advantageous Effects of Invention

In the case where the X-ray opaque filler of the present invention or the dental X-ray opaque filler of the present invention is blended in a curable composition including a polymerizable monomer, the X-ray opaque filler of the present invention or the dental X-ray opaque filler of the present invention hardly reduces the transparency of a cured body even when the blending amount thereof is increased, unlike the related-art X-ray opaque filler formed of a rare earth metal fluoride. Accordingly, the use of the dental curable composition of the present invention including the X-ray opaque filler of the present invention enables treatment which is excellent in aesthetics and in which a treatment site can be easily recognized with a radiograph or the like. In addition, the X-ray opaque filler of the present invention can achieve both the transparency and the X-ray opacity of a cured body in polymerization-curable compositions for various applications, such as an adhesive and a paint, as well as dental applications. Further, according to the production method of the present invention, the X-ray opaque filler of the present invention having such excellent features as described above can be efficiently produced through use of an easily available material.

Brief Description of Drawings

FIG. 1 is a graph showing relationships between the maximum peak half width of crystalline rare earth metal fluoride particles in an X-ray opaque filler and the contrast ratio of a cured body in Examples 1 to 11 and Comparative Examples 1 to 4.
FIG. 2 is a graph showing relationships between the mechanochemical treatment time of raw material powder (crystalline rare earth metal fluoride particles) used for the production of the X-ray opaque filler and the maximum peak half width of the crystalline rare earth metal fluoride particles in the X-ray opaque filler in Examples 1 to 11 and Comparative Examples 1 to 4.
FIG. 3 is a graph showing relationships between the mechanochemical treatment time of the raw material powder (crystalline rare earth metal fluoride particles) used for the production of the X-ray opaque filler and the contrast ratio of the cured body in Examples 1 to 11 and Comparative Examples 1 to 4.

FIG. 4 is a graph showing relationships between the mechanochemical treatment time of the raw material powder (crystalline rare earth metal fluoride particles) used for the production of the X-ray opaque filler and the average primary particle diameter of the crystalline rare earth metal fluoride particles in the X-ray opaque filler in Examples 1 to 11 and Comparative Examples 1 to 4.

FIG. 5 is a graph showing relationships between the refractive index of a polymerizable monomer or a cured body of the polymerizable monomer and the contrast ratio of a curable composition or a cured body in Examples 16 to 22 and Comparative Examples 7 to 13.

FIG. 6 is a graph showing relationships between the refractive index of a polymerizable monomer or a cured body of the polymerizable monomer and the contrast ratio of a curable composition or a cured body in Examples 23 to 29 and Comparative Examples 14 to 20.

FIG. 7 is a graph showing relationships between the refractive index of a polymerizable monomer or a cured body of the polymerizable monomer and the contrast ratio of a curable composition or a cured body in Examples 30 to 36 and Comparative Examples 21 to 27.

FIG. 8 is a graph showing relationships between the refractive index of a polymerizable monomer or a cured body of the polymerizable monomer and the contrast ratio of a curable composition or a cured body in Examples 37 to 43 and Comparative Examples 28 to 34.

Description of Embodiments

The inventors of the present invention have made extensive investigations in order to solve the above-mentioned problem of the related-art X-ray opaque filler formed of a rare earth metal fluoride, that is, a problem in that when the related-art X-ray opaque filler is blended in a curable composition including a polymerizable monomer, the transparency of a cured body is significantly reduced along with an increase in blending amount of the filler. As a result, the inventors have accidentally found that, in the case of using powder obtained by subjecting crystalline ytterbium fluoride known as an X-ray opaque filler to mechanochemical treatment with a wet bead mill device for a long period of time, the transparency of the cured body is hardly reduced even when the blending amount of the filler is increased. Moreover, the inventors have made further investigations based on the finding, and as a result, have found the following facts (i) and (ii), and thus completed the present invention.

(i) The crystallinity of crystal particles is reduced through the mechanochemical treatment.
(ii) The crystallinity of the crystal particles is grasped by the full width at half maximum of the maximum intensity peak (maximum peak half width) derived from the crystalline ytterbium fluoride in a diffraction pattern obtained through X-ray diffraction measurement of the powder. Moreover, even under the state in which a reduction in size of the powder hardly occurs, when the degree of a reduction in crystallinity exceeds a certain extent, a preventing effect on a reduction in transparency is obtained.

The reason for expression of the above-mentioned effect of the X-ray opaque filler of the present invention, that is, such an effect that the transparency of a cured body is hardly reduced even when the blending amount of the X-ray opaque filler in a curable composition is increased, is not entirely clear. In addition, the present invention is by no means bound by any theory. However, based on the following facts (1) to (5) found from the investigations made by the inventors, the inventors have presumed the reason to be as described below.

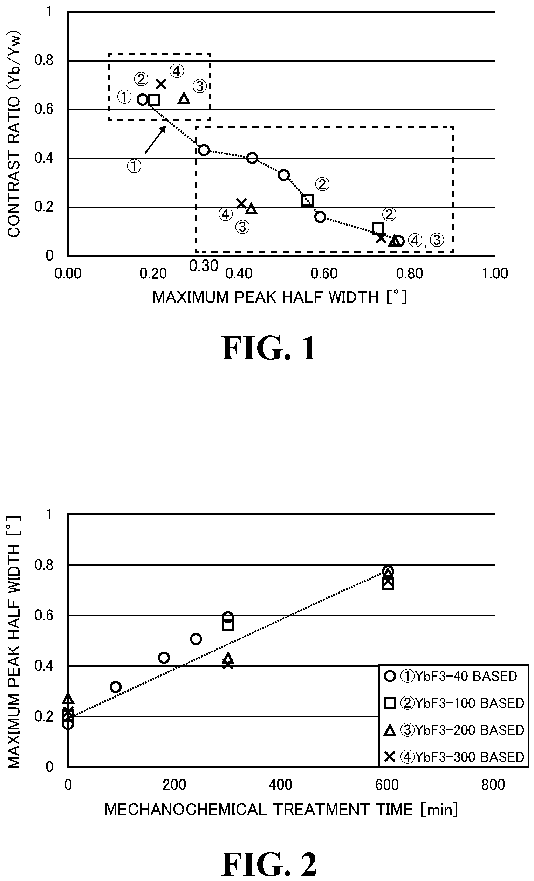

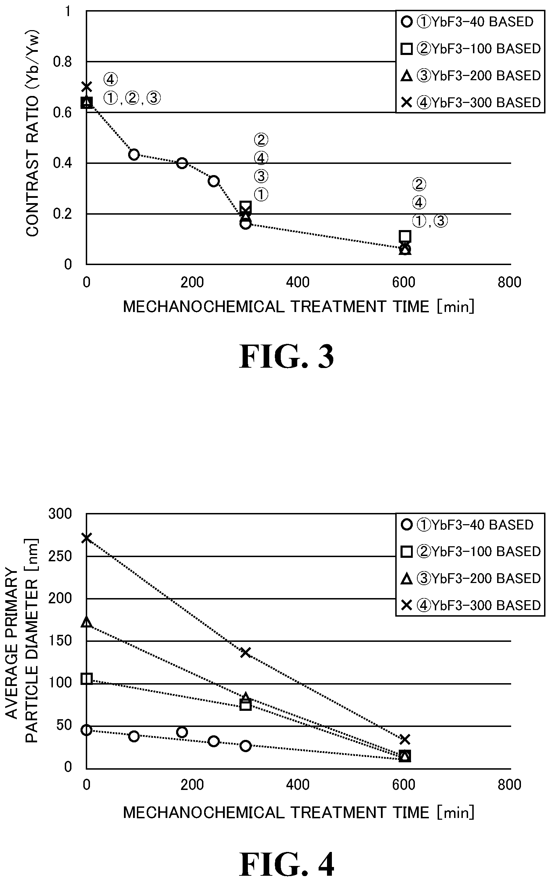

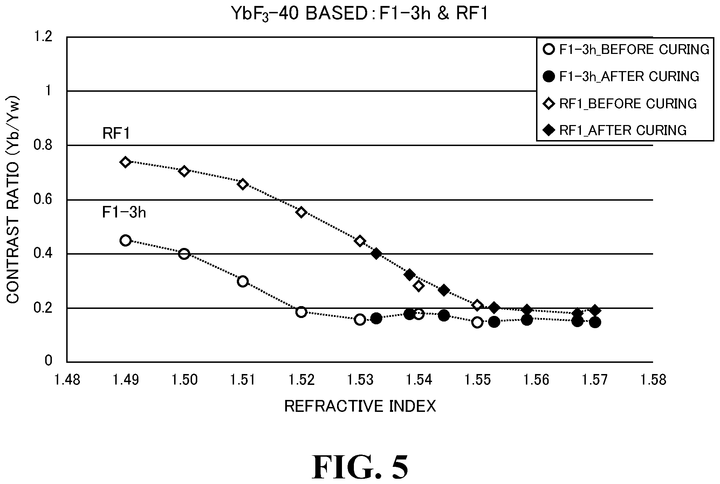

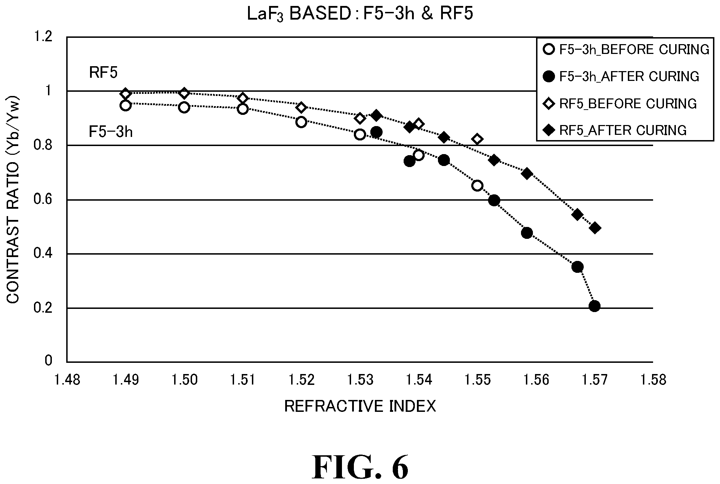

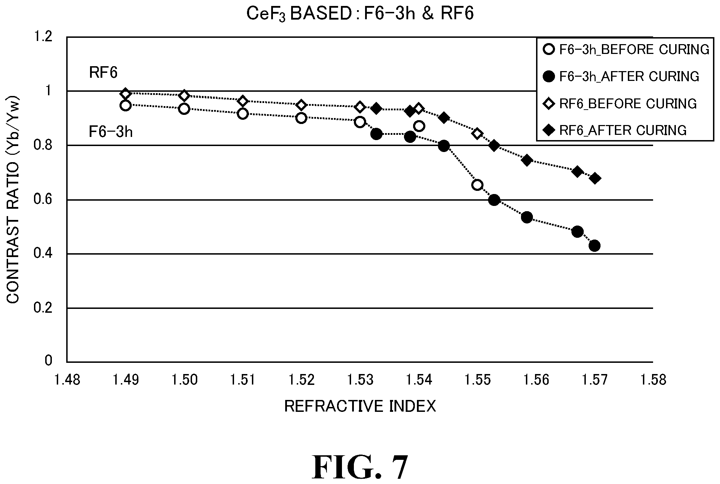

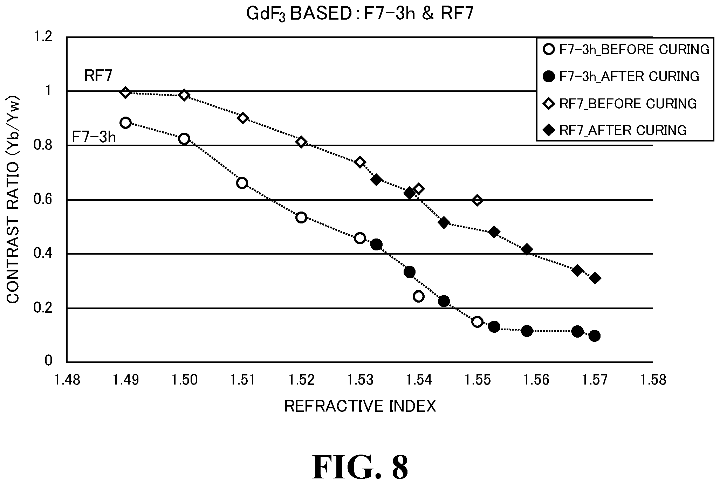

(1) There was a relatively clear correlation between the maximum peak half width and the transparency (contrast ratio) of the cured body (see FIG. 1 described later).
(2) The maximum peak half width is increased almost in proportion to a mechanochemical treatment time (see FIG. 2 described later).
(3) There was no correlation between the average primary particle diameter of the powder and the transparency (contrast ratio) of the cured body (see FIG. 3 and FIG. 4 described later).
(4) The preventing effect on a reduction in transparency was not only exhibited when the refractive index of a matrix resin (a cured body of a polymerizable monomer) was a specific value, and the effect was exhibited for a matrix resin having a refractive index in a relatively wide range (see FIG. 5 to FIG. 8 described later).
(5) In association with the above-mentioned fact (4), (while the polymerizable monomer and the cured body thereof had different refractive indices), the preventing effect on a reduction in transparency was exhibited for both the curable composition in a paste state before curing and the cured body after curing (see FIG. 5 to FIG. 8 described later).

That is, the inventors have presumed the reason for expression of the effect of the X-ray opaque filler of the present invention to be as described below. First, it is conceived that, in a system in which inorganic fine particles are dispersed in a resin matrix, a reduction in transparency is largely affected by diffuse reflection of light at an interface between each of the particles and the resin matrix. Meanwhile, in each of crystalline rare earth metal fluoride particles, a vicinity of the surface thereof is gradually amorphized from the surface to the inside thereof through the mechanochemical treatment. As a result, a layer in which a refractive index is gradually reduced at a certain gradient from the inside to the surface (hereinafter also referred to as "refractive index gradient layer") is formed in the vicinity of the surface of each of the crystalline rare earth metal fluoride particles. Moreover, the refractive index gradient layer formed includes a portion having a refractive index consistent with the refractive index of the resin matrix. In this case, the ratio of reflected light is reduced (the ratio of transmitted light is increased), and a reduction in transparency is suppressed.

Now, the present invention is described in detail. The expression "(from) x to y" using numerical values "x" and "y" as used herein means "x or more and y or less" unless otherwise specified. When a unit is assigned only to the numerical value "y" in the expression, the unit is also applied to the numerical value "x". In addition, the term "(meth)acrylic" as used herein means both "acrylic" and "methacrylic". Similarly, the term "(meth)acrylate" means both "acrylate" and "methacrylate", and the term "(meth)acryloyl" means both "acryloyl" and "methacryloyl".

1. With Regard to X-ray Opaque Filler of the Present Invention

The X-ray opaque filler of the present invention is an X-ray opaque filler that is blended in a curable composition including a polymerizable monomer to impart X-ray opacity to the curable composition and a cured body thereof. The polymerizable monomer in the curable composition to which the X-ray opacity is imparted is not particularly limited as long as the polymerizable monomer is a compound having polymerizability, and a generally used compound may be used depending on the applications. For example, when the curable composition is a dental curable composition, a radical polymerizable monomer or the like, which is versatilely used in this application, may be used. From the viewpoint of transparency, the polymerizable monomer to be used in the curable composition in which the X-ray opaque filler of the present invention is to be blended is preferably a polymerizable monomer that satisfies the conditions in which the difference in refractive index "$n_X$-$n_M$" falls within a specific range. Herein, $n_X$ represents the refractive index of crystalline rare earth metal fluoride particles serving as a main component of the X-ray opaque filler of the present invention, and $n_M$ represents the refractive index of a cured body of the polymerizable monomer. Specifically, the following expression (1) is preferably satisfied, the following expression (2) is more preferably satisfied, and the following expression (3) is most preferably satisfied. As described above, the crystalline rare earth metal fluoride particles having been subjected to mechanochemical treatment are each presumed to have a refractive index gradient layer in the vicinity of the surface. However, the ratio of the refractive index gradient layer in the entirety of each of the crystalline rare earth metal fluoride particles having been subjected to the mechanochemical treatment is significantly small, and hence it is conceived that the presence or absence of the refractive index gradient layer does not substantially affect the refractive index of the entirety of the particles. In addition, the inventors have recognized that there is no substantial significant difference between the refractive indices of the crystalline rare earth metal fluoride particles before and after the mechanochemical treatment. Accordingly, whether the crystalline rare earth metal fluoride particles serving as a main component of the X-ray opaque filler were subjected to the mechanochemical treatment or not, a value obtained by measuring the refractive index of the crystalline rare earth metal fluoride particles before the mechanochemical treatment was used as the refractive index $n_X$ for convenience in the calculation of the difference in refractive index "$n_X$-$n_M$" in each of the expressions (1) to (3).

$$\text{Expression (1)} \quad -0.02 \leq (n_X - n_M) \leq 0.1$$

$$\text{Expression (2)} \quad -0.01 \leq (n_X - n_M) \leq 0.07$$

$$\text{Expression (3)} \quad 0 \leq (n_X - n_M) \leq 0.05$$

The X-ray opaque filler of the present invention is required to be formed of any powder selected from the group consisting of: first powder including crystalline rare earth metal fluoride particles as a main component and having a maximum peak half width of 0.3° or more in an X-ray diffraction pattern; and second powder obtained by subjecting the first powder to surface treatment. Even in the case where the powder includes the crystalline rare earth metal fluoride particles as a main component, when the powder has a maximum peak half width of less than 0.3° in the X-ray diffraction pattern, it becomes difficult to obtain the preventing effect on a reduction in transparency. In the following description, the powder forming the X-ray opaque filler of the present invention is simply referred to as "powder" when the first powder and the second powder are not particularly distinguished from each other.

For example, (i) a substance derived from a surface treatment agent such as a silane coupling agent, or (ii) a substance derived from a coating agent such as silica, a surface treatment agent such as a silane coupling agent, or any other optional trace additive used in raw material powder in a production method of the present invention described later corresponds to a component other than the crystalline rare earth metal fluoride particles. In addition, the phrase "including crystalline rare earth metal fluoride particles as a main component" means that 85 mass% or more of the total mass of the powder is formed of the crystalline rare earth metal fluoride particles. In this case, 90 mass% or more of the total mass of the powder is preferably formed of the crystalline rare earth metal fluoride particles.

Now, the crystalline rare earth metal fluoride particles serving as a main component of the powder and the maximum peak half width are described in detail.

1-1. With Regard to Crystalline Rare Earth Metal Fluoride Particles

As the rare earth metal fluoride in the crystalline rare earth metal fluoride particles, lanthanum fluoride ($LaF_3$), cerium fluoride ($CeF_3$), ytterbium fluoride ($YbF_3$), or gadolinium fluoride ($GdF_3$) is preferably used because of its color tone or safety, and ytterbium fluoride ($YbF_3$) is most preferably used from the viewpoint of its X-ray opacity. The crystal structures of the crystalline rare earth metal fluoride particles are not particularly limited. In general, particles having stable crystal structures at normal temperature and normal pressure are used depending on the kind of the rare earth metal fluoride. Those crystalline rare earth metal fluorides each generally have a refractive index for a sodium d line at 25°C in the range of from 1.50 to 1.65.

From the viewpoints of a maintaining effect on the transparency, and the glossiness of a cured body at the time of blending in a dental curable composition, the first powder preferably includes, as a main component, crystalline rare earth metal fluoride particles having an average primary particle diameter of from 1 nm to 500 nm measured through observation with an electron microscope and/or aggregated particles thereof. In this case, the crystalline rare earth metal fluoride particles particularly preferably have an average primary particle diameter of from 5 nm to 300 nm. Herein, the primary particle diameter measured through observation with an electron microscope means the following value: in an observation image obtained through observation with a scanning electron microscope (SEM) at a magnification of 100,000 times, the particle diameters of 100 primary particles are determined, and an average value thereof is obtained.

In addition, from the viewpoint of easy handleability, an average particle diameter (of the entire powder including the aggregated particles) measured by a laser diffraction scattering method is preferably from 0.1 $\mu$m to 0.6 um, particularly preferably from 0.1 um to 0.3 $\mu$m.

1-2. With Regard to Maximum Peak Half Width

In order to obtain a preventing effect on a reduction in transparency, the X-ray opaque filler of the present invention is required to have a maximum peak half width, that is, a full width at half maximum of a maximum intensity peak derived from the crystalline rare earth metal fluoride particles of 0.3° or more in the X-ray diffraction pattern of the powder. From the viewpoint of the magnitude of the preventing effect on a reduction in transparency, the maximum peak half width is preferably 0.4° or more, particularly preferably 0.5° or more. The upper limit value of the maximum peak half width is not particularly limited, but in general, does not exceed 40°. However, based on Table 1 described later, from the viewpoint of the productivity (mechanochemical treatment time) of the X-ray opaque filler of this embodiment, the maximum peak half width is preferably 0.77° or less. In addition, from the viewpoint of more satisfactorily balancing the transparency of a cured body and the productivity of the X-ray opaque filler, the maximum peak half width is preferably from 0.47° to 0.68°, more preferably from 0.51° to 0.59°.

In the present invention, the maximum peak half width may be determined through X-ray diffraction measurement of the powder serving as the X-ray opaque filler of the present invention. Specifically, X-ray diffraction measurement is performed on a measurement sample (powder) in the range of 2Θ of from 20° to 120° with an X-ray diffractometer to provide an X-ray diffraction pattern (chart) in which the abscissa shows 2Θ (°) and the ordinate shows a diffraction intensity. With the chart, peaks derived from the crystalline rare earth metal fluoride particles are identified, and a peak having the maximum intensity out of the peaks is specified. For example, when the material of the crystalline rare earth metal fluoride particles is $YbF_3$, a peak corresponding to a crystal plane (111), which appears around 2θ=28.0°, is the peak having the maximum intensity. Subsequently, in the peak having the maximum intensity, a peak width at an intensity of 50% of the maximum intensity (50% intensity) is obtained as the maximum peak half width. Herein, the peak width is an absolute value (unit: "deg [°]") of a difference in 2Θ between two intersection points at which a line that is parallel to the abscissa of the X-ray diffraction pattern (chart) and is positioned at the 50% intensity intersects with a peak line. In the measurement, the powder from which coarse particles have been removed with, for example, a sieve having an opening of 100 um is preferably used as the measurement sample.

In general, a correlation known as the Scherrer equation is established between the full width at half maximum of a diffraction peak in X-ray diffraction and a crystallite size, and it is known that the crystallite size is in inverse proportion to the full width at half maximum. In addition, the full width at half maximum is also affected by the strain of a crystal lattice, and the full width at half maximum tends to be expanded when the strain of the crystal lattice is increased. It is conceived that when the strain of the crystal lattice is increased and the crystallite size is reduced to cause fine crystallites to orient in various directions, amorphous properties are increased. Accordingly, the maximum peak half width can be said to be an indicator of the crystallinity of the rare earth metal fluoride.

As described later, a rare earth metal fluoride-based X-ray opaque filler that has hitherto been generally used and a (crystalline) rare earth metal fluoride available as a reagent were each subjected to X-ray diffraction measurement, and as a result, were each found to have a maximum peak half width of less than 0.3° (from 0.17° to 0.27°). From this result, it can be said that the average crystallite size of the crystalline rare earth metal fluoride particles forming the X-ray opaque filler of the present invention is significantly reduced, and the crystallinity thereof is slightly reduced as a whole.

According to the investigations made by the inventors, based on the fact (i) that the above-mentioned facts (1) to (5) are recognized, and the fact (ii) that, even in a system in which a primary particle diameter is originally small and the primary particle diameter is hardly changed even when a treatment time is prolonged, the maximum peak half width is increased through the mechanochemical treatment in the production method of the present invention in accordance with the length of the treatment time, it is presumably conceived that the crystallinity of the particles is heterogeneous. However, for the individual crystalline rare earth metal fluoride particles forming the powder serving as the X-ray opaque filler of the present invention, it is difficult and substantially impossible to analyze the state of its crystallite and the state of the strain of its crystal lattice. In view of the foregoing, in the present invention, the X-ray opaque filler of the present invention is specified by using the maximum peak half width as an indicator of averaged crystallinity. In addition, under such circumstances, the X-ray opaque filler of the present invention can also be said to be an X-ray opaque filler obtained by the production method of the present invention.

## 2. With Regard to Production Method of the Present Invention

The production method of the present invention is a method of producing the X-ray opaque filler of the present invention, including a step of subjecting raw material powder including crystalline rare earth metal fluoride particles as a main component and having a maximum peak half width of less than 0.3° to mechanochemical treatment so that the maximum peak half width becomes 0.3° or more.

As described above, a rare earth metal fluoride-based X-ray opaque filler that has hitherto been generally used and (crystalline) rare earth metal fluoride powder available as a reagent each generally have a maximum peak half width of less than 0.3°. Accordingly, such powder may be used as the raw material powder without particular limitation. When concern is raised that the raw material powder has low crystallinity, it is preferred to use the raw material powder after subjecting the raw material powder to X-ray diffraction measurement to recognize that the maximum peak half width is less than 0.3°. Commercially available crystalline rare earth metal fluoride powders for X-ray opaque fillers also include powder whose surface is coated with nano silica and powder subjected to surface treatment with a silane coupling agent or the like. In the production method of the present invention, those powders may each be used as it is as the raw material powder. In the mechanochemical treatment described later, depending on conditions, when the treatment time of the raw material powder is prolonged, pulverization of the particles occurs to cause disintegration of secondary particles (aggregated particles) or primary particles, to thereby reduce the particle diameter of the raw material powder. However, through mechanochemical treatment for about several hours, the particle diameter of the raw material powder is not significantly changed. From this fact, as the particle diameter of the raw material powder, its average primary particle diameter measured through observation with an electron microscope (in the same manner as in the case of the powder forming the X-ray opaque filler of the present invention) is preferably from 1 nm to 500 nm, particularly preferably from 5 nm to 300 nm, and its average particle diameter measured by a laser diffraction scattering method is preferably from 0.1 $\mu$m to 0.6 $\mu$m, particularly preferably from 0.1 $\mu$m to 0.3 $\mu$m.

In the production method of the present invention, the raw material powder is subjected to the mechanochemical treatment so that the maximum peak half width becomes 0.3° or more. Herein, the mechanochemical treatment means treatment in which mechanical energy is applied to the raw material powder, and also means treatment in which at least one of mechanical frictional crushing, pulverization, or dispersion is performed. A wet method is preferably adopted as a method for the mechanochemical treatment because the crystallinity of the crystalline rare earth metal fluoride powder (or particles) can be reliably and efficiently controlled to desired crystallinity. A treatment method using a wet bead mill is particularly preferred. When the mechanochemical treatment is performed by a wet method, a medium including a solvent, such as water or an alcohol, or the polymerizable monomer may be used as a medium. However, from the viewpoints of the dispersibility of the X-ray opaque filler and addition to a dental composition, the medium is preferably a medium in a liquid form at normal temperature (from 15°C to 25°C) .

Now, the production method of the present invention is described in detail by taking treatment using a wet bead mill as an example.

In the mechanochemical treatment using a wet bead mill, a slurry obtained by mixing the raw material powder to be subjected to the mechanochemical treatment and a medium is brought into contact with media (beads) to which movement is applied through stirring, vibration, or the like. Thus, the raw material powder is pulverized and disintegrated. Examples of the material of the beads used as the media include glass, alumina, zircon, zirconia, steel, and a resin. Of those, alumina or zirconia is preferred because these materials are each excellent in abrasion resistance and each have relatively low contamination. The sizes of the beads to be used may be selected depending on the target particle diameter of the X-ray opaque filler without particular limitation, but generally, beads each having a diameter of from 0.01 mm to 0.5 mm are preferably used. Thus, an X-ray opaque filler having a particle diameter preferred for addition to a dental curable composition can be obtained.

Examples of the wet bead mill include, according to its operation mode: a batch mode in which treatment is performed by directly loading the slurry and the beads in a device; a circulation mode in which the slurry is circulated between a tank and a device; and a pass mode in which the slurry passes through a device at predetermined times. Those operation modes may be selected depending on the amount of the raw material powder used for the mechanochemical treatment. A bead mill of a circulation mode is preferably used because the bead mill has satisfactory productivity and can treat the raw material powder in a relatively large amount.

Depending on the operation mode, such as the circulation mode or the pass mode, the slurry and the beads are required to be separated from each other at the time of mechanochemical treatment. A bead separation mode is, for example, a slit mode, a screen mode, or a centrifugal separation mode. Those bead separation modes may be selected depending on the particle diameters of the beads to be used, and any of those modes may be used without particular limitation. The concentration of the slurry to be used in the mechanochemical treatment is preferably as follows: the amount of the raw material powder is 50 parts by mass or less with respect to 100 parts by mass of the medium. When the amount of the raw material powder in the slurry is more than 50 parts by mass, the viscosity of the slurry is increased, and the mechanochemical treatment may become difficult.

An increase in viscosity of the slurry can be suppressed by adding a dispersant to the slurry. Accordingly, when the dispersant is added to the slurry, a slurry having a higher concentration can be subjected to the mechanochemical treatment. Any known surfactant to be used for general dispersion treatment of a filler may be used as the dispersant to be used without particular limitation, and examples thereof include a nonionic surfactant, an anionic surfactant, a cationic surfactant, an ampholytic surfactant, and a polymer-based surfactant thereof. Specific examples thereof include a glycerin fatty acid ester and an alkylene glycol adduct thereof, an aliphatic monocarboxylic acid salt, an alkyl amine salt, and an alkyl betaine. When a dental curable composition is prepared by using the raw material powder having been subjected to the mechanochemical treatment (crystalline rare earth metal fluoride particles having a maximum peak half width of 0.3° or more), a cationic surfactant is preferably used as the dispersant from the viewpoint of the dispersibility of the raw material powder after the mechanochemical treatment in the dental curable composition.

The conditions of the mechanochemical treatment vary depending on conditions, such as the operation mode of the wet bead mill device to be used, the diameters of the beads, the maximum peak half width of the raw material powder, and the concentration of the slurry. Those conditions may each be determined by performing a preliminary experiment with a device in which the mechanochemical treatment is actually performed, and checking the maximum peak half width of the raw material powder after the mechanochemical treatment with respect to a mechanochemical treatment time. In addition, at the time of production of the X-ray opaque filler, the maximum peak half width is appropriately checked by sampling a treatment slurry as required. Thus, an X-ray opaque filler including, as a main component, crystalline rare earth metal fluoride particles reliably having a desired maximum peak half width can be produced.

The raw material powder having been subjected to the mechanochemical treatment to have a maximum peak half width of 0.3° or more (crystalline rare earth metal fluoride particles having been subjected to the mechanochemical treatment) is generally subjected to an operation, such as concentration, drying, or filtration. Thus, the X-ray opaque filler of the present invention is obtained. When the polymerizable monomer is used as the medium in wet treatment, the raw material powder having been subjected to the mechanochemical treatment may be used as it is without performance of those operations. In addition, the resultant X-ray opaque filler may be subjected to surface treatment for improving an affinity for various polymerizable monomers and polymers thereof. A compound, such as a silane coupling agent or a titanate coupling agent, which is generally used, may be used as a surface treatment agent.

3. With Regard to Dental Curable Composition of the Present Invention

As described above, the X-ray opaque filler of the present invention is particularly useful as a filler to be blended in a dental curable composition (that is, a dental X-ray opaque filler). The dental curable composition has blended therein, in addition to the X-ray opaque filler of the present invention, a polymerizable monomer and a polymerization initiator.

Any known polymerizable monomer to be used for this application may be used as the polymerizable monomer without limitation. Specific examples thereof may include (meth)acrylate-based monomers, such as methyl (meth)acrylate, glycidyl (meth)acrylate, 2-cyanomethyl (meth)acrylate, polyethylene glycol mono(meth)acrylate, allyl (meth)acrylate, 2-hydroxyethyl mono(meth)acrylate, ethylene glycol di(meth)acrylate, diethylene glycol di(meth)acrylate, triethylene glycol di(meth)acrylate, nonaethylene glycol di(meth)acrylate, propylene glycol di(meth)acrylate, dipropylene glycol di(meth)acrylate, 2,2-bis[4-(meth)acryloyloxyethoxyphenyl]propane, 2,2-bis[4-(meth)acryloyloxyethoxyethoxyphenyl]propane, 2,2-bis{4-[3-(meth)acryloyloxy-2-hydroxypropoxy]phenyl}propane, 1,4-butanediol di(meth)acrylate, 1,3-hexanediol di(meth)acrylate, urethane di(meth)acrylate, and trimethylolpropane di(meth)acrylate.

The polymerizable monomers may be used alone or in combination thereof. However, as described above, from the viewpoint of the transparency of a cured body to be obtained, the polymerizable monomer is preferably a polymerizable monomer that satisfies the conditions in which the difference in refractive index "$n_X-n_M$" falls within a specific range as described above. Specifically, the polymerizable monomer is preferably a polymerizable monomer that satisfies the expression (1), more preferably a polymerizable monomer that satisfies the expression (2), most preferably a polymerizable monomer that satisfies the expression (3). The refractive index of the cured body of the polymerizable monomer may be adjusted by combining a plurality of polymerizable monomers, and the above-mentioned known polymerizable monomers may be mixed at an appropriate ratio and used.

The blending amount of the X-ray opaque filler of the present invention to be blended in the dental curable composition of the present invention is not particularly limited as long as the dental curable composition is in a paste form, but generally falls within the range of preferably from 1 part by mass to 80 parts by mass, more preferably from 3 parts by mass to 70 parts by mass with respect to 100 parts by mass of the dental curable composition. Further, from the viewpoints of imparting X-ray opacity to a cured body and various physical properties (e.g., mechanical strength and hardness), the blending amount of the X-ray opaque filler is still more preferably from 10 parts by mass to 40 parts by mass with respect to 100 parts by mass of the dental curable composition. In addition, from the viewpoint of imparting X-ray opacity to the cured body, it is also preferred to blend 1 part by mass to 400 parts by mass of the X-ray opaque filler with 100 parts by mass of the polymerizable monomer.

For example, any chemical polymerization initiator, photopolymerization initiator, or thermal polymerization initiator used as a polymerization initiator capable of polymerizing the polymerizable monomer may be used as the polymerization initiator without particular limitation.

The blending amount of the polymerization initiator is not particularly limited as long as the polymerization can be initiated, but generally falls within the range of from 0.001 part by mass to 10 parts by mass with respect to 100 parts by mass of the polymerizable monomer. From the viewpoints of a polymerization rate and various physical properties (e.g., weather resistance and hardness) of the cured body to be obtained, it is preferred to blend 0.05 part by mass to 5 parts by mass of the polymerization initiator on the above-mentioned basis.

The kind of the polymerization initiator may be selected depending on the applications of the dental curable composition. When the dental curable composition is a dental filling restoration material that is cured in an oral cavity, a photopolymerization initiator is preferably used. In addition, when the dental curable composition is turned into a mill blank in which a bulk body obtained by preliminarily curing the dental curable composition is utilized through cutting in a dental office, a dental laboratory, or the like, a thermal polymerization initiator is preferably used.

Examples of the photopolymerization initiator include benzoin alkyl ethers, benzyl ketals, benzophenones, α-diketones, thioxanthone compounds, and bisacyl phosphine oxides. A reducing agent is often added to the photopolymerization initiator. Examples of the reducing agent include aromatic amines, aliphatic amines, aldehydes, and sulfur-containing compounds. Further, a trihalomethyl triazine compound, an aryliodonium salt, or the like may be added thereto as required.

The dental curable composition of the present invention may have blended therein, in addition to the above-mentioned components, any other component that is known as a blending component in the dental curable composition, particularly a dental filling restoration material. Examples of such component include known additives, such as any other filler except the X-ray opaque filler of the present invention, a polymerization inhibitor, a UV absorber, a dye, an antistatic agent, a pigment, a fragrance, an organic solvent, and a thickener.

Any of an organic filler or an inorganic filler that is blended in the dental curable composition may be blended as the other filler. Examples of the organic filler include particles formed of organic polymers, such as polymethyl methacrylate, polyethyl methacrylate, a methyl methacrylate-ethyl methacrylate copolymer, cross-linked polymethyl methacrylate, cross-linked polyethyl methacrylate, an ethylenevinyl acetate copolymer, a styrene-butadiene copolymer, an acrylonitrile-styrene copolymer, and an acrylonitrile-butadiene-styrene copolymer.

Specific examples of the inorganic filler include inorganic particles, such as quartz, silica, alumina, silica titania, silica zirconia, lanthanum glass, barium glass, strontium glass, and metal oxides.

The particle diameter and shape of the other filler are not particularly limited, and spherical or irregular particles having an average particle diameter of from 0.001 μm to 100 μm, which are generally used as a dental material, may be appropriately used depending on the purposes. In addition, also the refractive index of the other filler is not particularly limited, and a filler having a refractive index in the range of from 1.4 to 2.6, which a filler of a general dental curable composition has, may be used without limitation.

When the other filler is blended in the dental curable composition of the present invention, also the blending amount of the other filler is not particularly limited as long as the dental curable composition is in a paste form. However, when the dental curable composition is used as a dental filling restoration material, the total amount of the X-ray opaque filler and the other filler is preferably from 25 parts by mass to 400 parts by mass, more preferably from 40 parts by mass to 250 parts by mass with respect to 100 parts by mass of the polymerizable monomer.

The curable composition having blended therein the X-ray opaque filler of the present invention may be used not only for the above-mentioned dental applications but also for an adhesive, a paint, an optical material, or the like, but is particularly suitably used as a dental filling restoration material.

A method of producing the dental curable composition of the present invention is not particularly limited, and any known method of producing a curable composition may be appropriately adopted. Specifically, the X-ray opaque filler, the polymerizable monomer, the polymerization initiator, and any other blending component to be blended as required forming the dental curable composition of the present invention may be weighed in predetermined amounts and mixed with one another (i) in a dark place in the case of a dental curable composition of a photopolymerization type, or (ii) under room temperature or low temperature in the case of a dental curable composition of a thermal polymerization type, to thereby prepare a dental curable composition in a paste form. The dental curable composition of the present invention thus produced is stored under light shielding or under room temperature or low temperature until use. In the case of a dental curable composition of a chemical polymerization type, the curable composition is produced and stored in the same manner as in the case of the above-mentioned dental curable composition of a photopolymerization type or a thermal polymerization type under the state in which two or more kinds of components that generate active species by being mixed are physically separated from each other.

Any known polymerization means may be appropriately adopted as means for curing the dental curable composition of the present invention according to a polymerization initiation mechanism of the polymerization initiator used. Specifically, as the curing means, for example, irradiation with light from a light source, such as a carbon arc, a xenon lamp, a metal halide lamp, a tungsten lump, a fluorescent lamp, sunlight, a helium cadmium laser, or an argon laser, or heating with a heat curing unit or the like, or a method including a combination thereof may be used without any limitation. When the dental curable composition is polymerized through irradiation with light, an irradiation time varies depending on the wavelength and intensity of a light source, and the shape and material of the cured body, and hence may be determined in advance through a preliminary experiment. However, in general, it is preferred to adjust the blending ratios of various components in the dental curable composition so that the irradiation time falls within the range of from about 5 seconds to about 60 seconds.

Examples

The present invention is specifically described below by way of Examples, but the present invention is by no means limited to these Examples.

First, substances used as raw materials of curable compositions prepared in Examples and Comparative Examples and abbreviations of the substances, and evaluation methods for the raw materials and the curable compositions prepared are described.

1. Substances and Abbreviations thereof

1-1. Polymerizable Monomer

UDMA: 1,6-bis(methacrylethyloxycarbonylamino)-2,2,4-trimethylhexane
3G: triethylene glycol dimethacrylate
BisGMA: 2,2-bis[4-[2-hydroxy-3-(methacryloyloxy)propyloxy]phenyl]propane
D-2.6E: 2,2-bis[4-(methacryloxyethoxy)phenyl]propane

1-2. Polymerization Initiator

CQ: camphorquinone (manufactured by Tokyo Chemical Industry Co., Ltd.)
DMBE: ethyl dimethylbenzoate (manufactured by Tokyo Chemical Industry Co., Ltd.)

1-3. Filler

(1) Crystalline Rare Earth Metal Fluoride Particles (Raw Material Powder)

The following crystalline rare earth metal fluoride particles were used as a raw material (raw material powder) of an X-ray opaque filler.

RF1: $YbF_3$-40 (ytterbium fluoride having an average primary particle diameter of 40 nm, an average secondary particle diameter of 0.6 um, and a refractive index of 1.55, manufactured by Sukgyung AT Co., Ltd.)
RF2: $YbF_3$-100 (ytterbium fluoride having an average primary particle diameter of 100 nm, an average secondary particle diameter of 0.6 um, and a refractive index of 1.55, manufactured by Sukgyung AT Co., Ltd.)
RF3: $YbF_3$-200 (ytterbium fluoride having an average primary particle diameter of 200 nm, an average secondary particle diameter of 0.6 um, and a refractive index of 1.55, manufactured by Treibacher Industrie AG)
RF4: $YbF_3$-300 (ytterbium fluoride having an average primary particle diameter of 300 nm, an average secondary particle diameter of 0.6 um, and a refractive index of 1.55, manufactured by Treibacher Industrie AG)
RF5: $LaF_3$ (lanthanum fluoride having an average primary particle diameter of 400 nm, an average secondary particle diameter of 0.6 um, and a refractive index of 1.58, manufactured by FUJIFILM Wako Pure Chemical Corporation)
RF6: $CeF_3$ (cerium fluoride having an average primary particle diameter of 350 nm, an average secondary particle diameter of 0.7 um, and a refractive index of 1.63, manufactured by FUJIFILM Wako Pure Chemical Corporation)
RF7: $GdF_3$ (gadolinium fluoride having an average primary particle diameter of 390 nm, an average secondary particle diameter of 0.6 $\mu$m, and a refractive index of 1.62, manufactured by FUJIFILM Wako Pure Chemical Corporation)

The average primary particle diameter, the average secondary particle diameter, and the refractive index are values determined based on the evaluation methods described later.

2. Various Evaluation Methods

2-1. Measurement of Average Primary Particle Diameter

The average primary particle diameter of crystalline rare earth metal fluoride particles forming an X-ray opaque filler was determined by the following procedure with a scanning electron microscope. First, a measurement sample was prepared by fixing the X-ray opaque filler to a sample stage with a carbon paste, and subjecting the X-ray opaque filler to conductive treatment (platinum vapor deposition). Next, the measurement sample was observed with an electron microscope (JSM-7800F PRIME, manufactured by JEOL Ltd.) at a magnification of 100,000 times. The average particle diameter of 100 primary particles in the observation image obtained was determined as the average primary particle diameter. In addition, also the average primary particle diameter of the raw material powder was determined by the same procedure.

2-2. Measurement of Average Secondary Particle Diameter

The average secondary particle diameter of crystalline rare earth metal fluoride particles forming an X-ray opaque filler was determined by the following procedure through particle size distribution measurement. First, a suspension in which 0.1 g of powder (X-ray opaque filler) was suspended in 10 mL of ion-exchanged water was prepared. Next, under the state in which the suspension was irradiated with an ultrasonic wave, particle size distribution measurement was performed with a particle size distribution analyzer (LS13-320, manufactured by Beckman Coulter, Inc.) to provide a volume-based particle size distribution. Moreover, a particle diameter (D50v value) at which the ratio of the particles integrated from small diameters reached 50% in the volume-based particle size distribution was used as the average secondary particle diameter of the crystalline rare earth metal fluoride particles forming the X-ray opaque filler. In addition, also the average secondary particle diameter of the raw material powder was determined by the same procedure.

2-3. Measurement of Refractive Index

2-3-1. Refractive Index $n_0$ of Polymerizable Monomer

The refractive index $n_0$ of a polymerizable monomer used in the preparation of a curable composition was measured as a refractive index for a sodium d line at 25°C with Abbe Refractometer (DR-A1-Plus, manufactured by ATAGO Co., Ltd.).

2-3-2. Refractive Index $n_M$ of Polymer (Cured Body of Polymerizable Monomer)

A polymerizable monomer used in the preparation of a curable composition (provided that the polymerizable monomer included trace amounts of polymerization initiators (0.2 wt% of camphor quinone and 0.35 wt% of ethyl N,N-dimethyl-p-benzoate) for curing treatment) was filled in a through hole (diameter: 7 mm, through hole length: 0.5 mm) formed in a mold, and the through hole was then sealed while a polypropylene film was brought into pressure contact with both-side openings of the through hole. After that, the polymerizable monomer filled in the through hole was cured by being irradiated with light with a halogen-type dental light irradiator (Demetron LC, manufactured by Sybron) at a light amount of 500 mW/cm$^2$ for 30 seconds. After that, the refractive index $n_M$ of a cured body of the polymerizable monomer removed from the mold was measured by the same procedure as in the section 2-3-1.

2-3-3. Refractive Index $n_X$ of Crystalline Rare Earth Fluoride Particles

In a constant-temperature room at 25°C, 1 g of crystalline rare earth fluoride particles were suspended in 50 mL of anhydrous toluene in a 100 mL sample bottle. While the suspension was stirred with a stirrer, 1-bromotoluene was dropped thereinto little by little. The refractive index of the suspension at the time point when the suspension became most transparent was measured by the same procedure as in the section 2-3-1, and the value obtained was used as the refractive index $n_X$ of the crystalline rare earth fluoride particles. As described above, the refractive index of the crystalline rare earth fluoride particles before mechanochemical treatment was used in place of the refractive index $n_X$ of the crystalline rare earth fluoride particles after mechanochemical treatment.

2-4. Evaluation of Transparency (Contrast Ratio) of Curable Composition and Cured Body

Curable compositions prepared in Examples and Comparative Examples were each filled in a mold made of polyacetal having an inner diameter of 0.7 cm and a depth of 0.1 cm. When the transparency of the curable composition was evaluated, this mold was used as a sample. In addition, when the transparency of a cured body was evaluated, a cured body obtained by filling the curable composition in the mold in the same manner as described above and then curing the curable composition through irradiation with light at an irradiation distance of 0.5 cm for 20 seconds with a dental light irradiator (TOKUSO POWER LIGHT, manufactured by Tokuyama Corporation) was used as a sample. The evaluation of the transparency was performed as follows: the samples were each measured for Y values under a black background and a white background with a colorimeter (SE 7700, manufactured by Nippon Denshoku Industries Co., Ltd.); and the transparency (contrast ratio: Yb/Yw) was calculated from the following equation.

Yb/Yw=Y value (Yb) under a black background/Y value (Yw) under a white background

3. Production of X-ray Opaque Filler

The crystalline rare earth metal fluoride particles ($YbF_3$-40, $YbF_3$-100, $YbF_3$-200, $YbF_3$-300, $LaF_3$, $CeF_3$, and $GdF_3$) listed as raw material powders in the section 1-3 (1), and powders obtained by subjecting these raw material powders to mechanochemical treatment were each used as an X-ray opaque filler. The abbreviation of the X-ray opaque filler used in each of Examples and Comparative Examples, the abbreviation of the crystalline rare earth metal fluoride particles (the raw material powder in itself or the powder obtained by subjecting the raw material powder to mechanochemical treatment) used in the production of the X-ray opaque filler, the mechanochemical treatment time of the raw material powder, the average primary particle diameter (in the case of the raw material powder having been subjected to mechanochemical treatment, a value after the mechanochemical treatment), the 2Θ and maximum peak half width of the maximum peak, and the refractive index $n_X$ are shown in Table 1.
The mechanochemical treatment was performed with a wet bead mill SC50 {manufactured by Mitsui Mining Co., Ltd.}. Moreover, a slurry obtained by mixing 5.0 parts by mass of the crystalline rare earth metal fluoride particles with 100 parts by mass of ion-exchanged water was subjected to dispersion treatment at a number of rotations of 3,000 rpm by using 100 g of zirconia beads of $\varphi$0.3 mm as media. The kind of the crystalline rare earth metal fluoride particles used in the dispersion treatment and a treatment time are shown in Table 1. In addition, for the measurement of the maximum peak half width, a measurement sample obtained by removing coarse particles from the raw material powder or the powder after the mechanochemical treatment with a sieve having an opening of 100 $\mu$m was used. Moreover, the measurement sample was loaded into a sample stage of an X-ray diffractometer {SmartLab, manufactured by Rigaku Corporation}, and was then subjected to X-ray diffraction measurement. Thus, an X-ray diffraction pattern (chart) in which the abscissa showed 2$\theta$ (°) and the ordinate showed a diffraction intensity was obtained. Herein, the 2$\Theta$ and the maximum peak half width (deg: °) shown in Table 1 are values about a peak of a crystal plane (111) in the X-ray diffraction pattern (chart).

Table 1

| X-ray opaque filler | Crystalline rare earth metal fluoride particles (raw material powder) | Mechanochemical treatment time [min] | Average primary particle diameter [nm] | Maximum peak: (111) | | Refractive index $n_X$ |
| | | | | 2$\theta$ [°] | Maximum peak half width [°] | |
| --- | --- | --- | --- | --- | --- | --- |
| RF1 | YbF$_3$-40 | 0 | 46 | 28.0 | 0.17 | 1.55 |
| RF2 | YbF$_3$-100 | | 106 | 28.0 | 0.20 | 1.55 |
| RF3 | YbF$_3$-200 | | 172 | 28.0 | 0.27 | 1.55 |
| RF4 | YbF$_3$-300 | | 270 | 27.9 | 0.22 | 1.55 |
| RF5 | LaF$_3$ | 0 | 403 | 27.7 | 0.12 | 1.58 |
| RF6 | CeF$_3$ | | 354 | 27.8 | 0.13 | 1.63 |
| RF7 | GdF$_3$ | | 389 | 27.6 | 0.17 | 1.62 |
| F1-1.5h | RF1: YbF$_3$-40 | 90 | 39 | 28.0 | 0.32 | 1.55 |
| F1-3h | | 180 | 44 | 27.9 | 0.43 | 1.55 |
| F1-4h | | 240 | 33 | 27.9 | 0.51 | 1.55 |
| F1-5h | | 300 | 28 | 27.8 | 0.59 | 1.55 |
| F1-10h | | 600 | 16 | 27.9 | 0.77 | 1.55 |
| F2-5h | RF2: YbF$_3$-100 | 300 | 76 | 28.0 | 0.56 | 1.55 |
| F2-10h | | 600 | 15 | 27.9 | 0.73 | 1.55 |
| F3-5h | RF3: YbF$_3$-200 | 300 | 84 | 27.9 | 0.43 | 1.55 |
| F3-10h | | 600 | 15 | 28.1 | 0.77 | 1.55 |
| F4-5h | RF4: YbF$_3$-300 | 300 | 136 | 28.0 | 0.41 | 1.55 |
| F4-10h | | 600 | 35 | 27.9 | 0.74 | 1.55 |
| F5-3h | RF5: LaF$_3$ | 180 | 273 | 27.7 | 0.41 | 1.58 |
| F6-3h | RF6: CeF$_3$ | 180 | 231 | 27.8 | 0.42 | 1.63 |
| F7-3h | RF7: GdF$_3$ | 180 | 256 | 27.6 | 0.58 | 1.62 |

4. Examples and Comparative Examples

Example 1

0.2 Part by weight of CQ and 0.35 part by weight of DMBE were added as polymerization initiators to 100 parts by mass of polymerizable monomers formed of 80 parts by mass of UDMA and 20 parts by mass of 3G, and the contents were stirred for 6 hours to prepare a liquid composition. F1 was added as an X-ray opaque filler to the liquid composition so that the amount of F1 was 150 parts by mass (60 wt%) with respect to 100 parts by mass of the polymerizable monomers, followed by mixing with an agate mortar. The resultant mixture was deaerated under a vacuum so that bubbles were removed therefrom. Thus, a curable composition in a paste form was obtained. The transparency (contrast ratio) of a cured body of the resultant curable composition was evaluated. The result was shown in Table 2.

Table 2

| No. | | X-ray opaque filler | Maximum peak half width [°] | Polymerizable monomer | | Cured body of curable composition | | |
|---|---|---|---|---|---|---|---|---|
| | | | | Kind | Mass ratio | Refractive index of cured body of polymerizable monomer ($n_M$) | Contrast ratio (Yb/Yw) | Difference in refractive index ($n_X$-$n_M$) |
| Example | 1 | F1-1.5h | 0.32 | UDMA/ 3G | 80/20 | 1.51 | 0.43 | 0.04 |
| | 2 | F1-3h | 0.43 | | | | 0.40 | 0.04 |
| | 3 | F1-4h | 0.51 | | | | 0.33 | 0.04 |
| | 4 | F1-5h | 0.59 | | | | 0.16 | 0.04 |
| | 5 | F2-5h | 0.56 | | | | 0.23 | 0.04 |
| | 6 | F3-5h | 0.43 | | | | 0.19 | 0.04 |
| | 7 | F4-5h | 0.41 | | | | 0.21 | 0.04 |
| | 8 | F1-10h | 0.77 | | | | 0.06 | 0.04 |
| | 9 | F2-10h | 0.73 | | | | 0.11 | 0.04 |
| | 10 | F3-10h | 0.77 | | | | 0.06 | 0.04 |
| | 11 | F4-10h | 0.74 | | | | 0.07 | 0.04 |
| | 12 | F1-3h | 0.43 | UDMA/ 3G | 50/50 | 1.51 | 0.39 | 0.04 |
| | 13 | | | | 20/80 | 1.51 | 0.40 | 0.04 |
| | 14 | F1-10h | 0.77 | | 50/50 | 1.51 | 0.06 | 0.04 |
| | 15 | | | | 20/80 | 1.51 | 0.07 | 0.04 |
| Comparative Example | 1 | RF1 | 0.17 | UDMA/ 3G | 80/20 | 1.51 | 0.64 | 0.04 |
| | 2 | RF2 | 0.20 | | | | 0.64 | 0.04 |
| | 3 | RF3 | 0.27 | | | | 0.65 | 0.04 |
| | 4 | RF4 | 0.22 | | | | 0.70 | 0.04 |
| | 5 | RF1 | 0.17 | UDMA/ 3G | 50/50 | 1.51 | 0.63 | 0.04 |
| | 6 | | | | 20/80 | 1.51 | 0.64 | 0.04 |

Examples 2 to 15 and Comparative Examples 1 to 6

Curable compositions of Examples 2 to 15 and Comparative Examples 1 to 6 were each prepared in the same manner as in Example 1 except that, in Example 1, the materials shown in Table 2 were used as the polymerizable monomers, the polymerization initiator, and the X-ray opaque filler to be blended. Moreover, the transparency (contrast ratio) of a cured body of the resultant curable composition was evaluated. The results were collectively shown in Table 2.

As shown in Table 2, in each of Comparative Examples 1 to 6, in which the X-ray opaque filler used had a maximum peak half width of less than 0.3°, the resultant cured body was relatively non-transparent (had a high contrast ratio of from 0.63 to 0.70). Meanwhile, in each of Examples 1 to 15, in which the X-ray opaque filler including the crystalline rare earth metal fluoride particles having been subjected to the mechanochemical treatment to have a maximum peak half width of 0.3° or more was used, the resultant cured body had satisfactory transparency (contrast ratio: from 0.06 to 0.43). In addition, as the value of the maximum peak half width became higher, the transparency of the cured body was increased more (the contrast ratio was reduced more).

Examples 16 to 22 and Comparative Examples 7 to 13

Curable compositions of Examples 16 to 22 were each prepared in the same manner as in Example 2 except that, in Example 2, the composition of the polymerizable monomer(s) to be blended was changed as shown in Table 3. In addition, curable compositions of Comparative Examples 7 to 13 were each prepared in the same manner as in Comparative Example 1 except that, in Comparative Example 1, the composition of the polymerizable monomer(s) to be blended was changed as shown in Table 3. Moreover, the transparency of each of the resultant curable composition in a paste form and a cured body thereof was evaluated. The results were collectively shown in Table 3.

Table 3

| No. | | X-ray opaque filler | Polymerizable monomer | | Curable composition | | | Cured body of curable composition | | |
|---|---|---|---|---|---|---|---|---|---|---|
| | | | Kind | Mass ratio | Refractive index of polymerizable monomer ($n_0$) | Contrast ratio (Yb/Yw) | Difference in refractive index ($n_X$-$n_0$) | Refractive index of cured body of polymerizable monomer ($n_X$) | Contrast ratio (Yb/Yw) | Difference in refractive index ($n_X$-$n_M$) |
| Example | 16 | F1-3h | D-2.6E/3G | 40/60 | 1.49 | 0.45 | 0.06 | 1.53 | 0.17 | 0.02 |
| | 17 | | | 50/50 | 1.50 | 0.40 | 0.05 | 1.54 | 0.18 | 0.01 |
| | 18 | | | 60/40 | 1.51 | 0.30 | 0.04 | 1.54 | 0.18 | 0.01 |
| | 19 | | | 75/25 | 1.52 | 0.19 | 0.03 | 1.55 | 0.15 | 0.00 |
| | 20 | | | 85/15 | 1.53 | 0.16 | 0.02 | 1.56 | 0.16 | -0.01 |
| | 21 | | D-2.6E | 100 | 1.54 | 0.18 | 0.01 | 1.57 | 0.16 | -0.02 |
| | 22 | | BisGMA | 100 | 1.55 | 0.15 | 0.00 | 1.57 | 0.15 | -0.02 |
| Comparative Example | 7 | RF1 | D-2.6E/3G | 40/60 | 1.49 | 0.74 | 0.06 | 1.53 | 0.40 | 0.02 |
| | 8 | | | 50/50 | 1.50 | 0.70 | 0.05 | 1.54 | 0.32 | 0.01 |
| | 9 | | | 60/40 | 1.51 | 0.66 | 0.04 | 1.54 | 0.27 | 0.01 |
| | 10 | | | 75/25 | 1.52 | 0.55 | 0.03 | 1.55 | 0.20 | 0.00 |
| | 11 | | | 85/15 | 1.53 | 0.45 | 0.02 | 1.56 | 0.19 | -0.01 |
| | 12 | | D-2.6E | 100 | 1.54 | 0.28 | 0.01 | 1.57 | 0.18 | -0.02 |
| | 13 | | BisGMA | 100 | 1.55 | 0.21 | 0.00 | 1.57 | 0.19 | -0.02 |

As shown in Table 3, in Examples (or Comparative Examples) using the same X-ray opaque filler, the refractive indices of the polymerizable monomer and the cured body of the polymerizable monomer vary depending on the composition of the polymerizable monomer(s). However, when Examples and Comparative Examples having the same composition of the polymerizable monomer(s) were compared to each other, it was recognized that the curable composition and the cured body each had a lower contrast ratio and thus had higher transparency in each of Examples 16 to 22, in which the X-ray opaque filler F1-3h having a maximum peak half width of 0.3° or more was used, than in each of Comparative Examples 7 to 13, in which the X-ray opaque filler RF1 having a maximum peak half width of less than 0.3° was used.

Examples 23 to 29 and Comparative Examples 14 to 20

Curable compositions of Examples 23 to 29 and Comparative Examples 14 to 20 were prepared in the same manner as in Examples 16 to 22 and Comparative Examples 7 to 13, respectively, except that the X-ray opaque filler used was changed to F5-3h ($LaF_3$) or RF5 ($LaF_3$). Moreover, the transparency of each of the resultant curable composition in a paste form and a cured body thereof was evaluated. The results were collectively shown in Table 4.

Table 4

| No. | | X-ray opaque filler | Polymerizable monomer | | Curable composition | | | Cured body of curable composition | | |
|---|---|---|---|---|---|---|---|---|---|---|
| | | | Kind | Mass ratio | Refractive index of polymerizable monomer ($n_0$) | Contrast ratio (Yb/Yw) | Difference in refractive index ($n_X - n_0$) | Refractive index of cured body of polymerizable monomer ($n_X$) | Contrast ratio (Yb/Yw) | Difference in refractive index ($n_X - n_M$) |
| Example | 23 | F5-3h (LaF$_3$) | D-2.6E/ 3G | 40/60 | 1.49 | 0.95 | 0.09 | 1.53 | 0.85 | 0.05 |
| | 24 | | | 50/50 | 1.50 | 0.94 | 0.08 | 1.54 | 0.74 | 0.04 |
| | 25 | | | 60/40 | 1.51 | 0.94 | 0.07 | 1.54 | 0.75 | 0.04 |
| | 26 | | | 75/25 | 1.52 | 0.89 | 0.06 | 1.55 | 0.60 | 0.03 |
| | 27 | | | 85/15 | 1.53 | 0.84 | 0.05 | 1.56 | 0.48 | 0.02 |
| | 28 | | D-2.6E | 100 | 1.54 | 0.77 | 0.04 | 1.57 | 0.35 | 0.01 |
| | 29 | | BisGMA | 100 | 1.55 | 0.65 | 0.03 | 1.57 | 0.21 | 0.01 |
| Comparative Example | 14 | RF5 (LaF$_3$) | D-2.6E/ 3G | 40/60 | 1.49 | 0.99 | 0.09 | 1.53 | 0.91 | 0.05 |
| | 15 | | | 50/50 | 1.50 | 0.99 | 0.08 | 1.54 | 0.87 | 0.04 |
| | 16 | | | 60/40 | 1.51 | 0.98 | 0.07 | 1.54 | 0.83 | 0.04 |
| | 17 | | | 75/25 | 1.52 | 0.94 | 0.06 | 1.55 | 0.75 | 0.03 |
| | 18 | | | 85/15 | 1.53 | 0.90 | 0.05 | 1.56 | 0.70 | 0.02 |
| | 19 | | D-2.6E | 100 | 1.54 | 0.88 | 0.04 | 1.57 | 0.55 | 0.01 |
| | 20 | | BisGMA | 100 | 1.55 | 0.82 | 0.03 | 1.57 | 0.50 | 0.01 |

As shown in Table 4, also in the $LaF_3$-based curable compositions, when Examples and Comparative Examples having the same composition of the polymerizable monomer(s) were compared to each other, it was recognized that both the curable composition and the cured body thereof had higher transparency (lower contrast ratio) in Examples.

Examples 30 to 36 and Comparative Examples 21 to 27

Curable compositions of Examples 30 to 36 and Comparative Examples 21 to 27 were prepared in the same manner as in Examples 16 to 22 and Comparative Examples 7 to 13, respectively, except that the X-ray opaque filler used was changed to F6-3h ($CeF_3$) or RF6 ($CeF_3$). Moreover, the transparency of each of the resultant curable composition in a paste form and a cured body thereof was evaluated. The results were collectively shown in Table 5.

Table 5

| No. | | X-ray opaque filler | Polymerizable monomer | | Curable composition | | | Cured body of curable composition | | |
|---|---|---|---|---|---|---|---|---|---|---|
| | | | Kind | Mass ratio | Refractive index of polymerizable monomer ($n_0$) | Contrast ratio (Yb/Yw) | Difference in refractive index ($n_X$-$n_0$) | Refractive index of cured body of polymerizable monomer ($n_X$) | Contrast ratio (Yb/Yw) | Difference in refractive index ($n_X$-$n_M$) |
| Example | 30 | F6-3h (CeF$_3$) | D-2.6E/ 3G | 40/60 | 1.49 | 0.95 | 0.14 | 1.53 | 0.85 | 0.10 |
| | 31 | | | 50/50 | 1.50 | 0.94 | 0.13 | 1.54 | 0.83 | 0.09 |
| | 32 | | | 60/40 | 1.51 | 0.92 | 0.12 | 1.54 | 0.80 | 0.09 |
| | 33 | | | 75/25 | 1.52 | 0.90 | 0.11 | 1.55 | 0.60 | 0.08 |
| | 34 | | | 85/15 | 1.53 | 0.89 | 0.10 | 1.56 | 0.54 | 0.07 |
| | 35 | | D-2.6E | 100 | 1.54 | 0.87 | 0.09 | 1.57 | 0.48 | 0.06 |
| | 36 | | BisGMA | 100 | 1.55 | 0.65 | 0.08 | 1.57 | 0.43 | 0.06 |
| Comparative Example | 21 | RF6 (CeF$_3$) | D-2.6E/ 3G | 40/60 | 1.49 | 0.99 | 0.14 | 1.53 | 0.94 | 0.10 |
| | 22 | | | 50/50 | 1.50 | 0.98 | 0.13 | 1.54 | 0.93 | 0.09 |
| | 23 | | | 60/40 | 1.51 | 0.96 | 0.12 | 1.54 | 0.90 | 0.09 |
| | 24 | | | 75/25 | 1.52 | 0.95 | 0.11 | 1.55 | 0.80 | 0.08 |
| | 25 | | | 85/15 | 1.53 | 0.94 | 0.10 | 1.56 | 0.75 | 0.07 |
| | 26 | | D-2.6E | 100 | 1.54 | 0.94 | 0.09 | 1.57 | 0.70 | 0.06 |
| | 27 | | BisGMA | 100 | 1.55 | 0.84 | 0.08 | 1.57 | 0.68 | 0.06 |

As shown in Table 5, also in the $CeF_3$-based curable compositions, when Examples and Comparative Examples having the same composition of the polymerizable monomer(s) were compared to each other, it was recognized that both the curable composition and the cured body thereof had higher transparency (lower contrast ratio) in Examples.

Examples 37 to 43 and Comparative Examples 28 to 34

Curable compositions of Examples 37 to 43 and Comparative Examples 28 to 34 were prepared in the same manner as in Examples 16 to 22 and Comparative Examples 7 to 13, respectively, except that the X-ray opaque filler used was changed to F7-3h ($GdF_3$) or RF7 ($GdF_3$). Moreover, the transparency of each of the resultant curable composition in a paste form and a cured body thereof was evaluated. The results were collectively shown in Table 6.

Table 6

| No. | | X-ray opaque filler | Polymerizable monomer | | Curable composition | | | Cured body of curable composition | | |
|---|---|---|---|---|---|---|---|---|---|---|
| | | | Kind | Mass ratio | Refractive index of polymerizable monomer ($n_0$) | Contrast ratio (Yb/Yw) | Difference in refractive index ($n_X$-$n_0$) | Refractive index of cured body of polymerizable monomer ($n_X$) | Contrast ratio (Yb/Yw) | Difference in refractive index ($n_X$-$n_M$) |
| Example | 37 | F7-3h (GdF$_3$) | D-2.6E/ 3G | 40/60 | 1.49 | 0.88 | 0.13 | 1.53 | 0.43 | 0.09 |
| | 38 | | | 50/50 | 1.50 | 0.82 | 0.12 | 1.54 | 0.33 | 0.08 |
| | 39 | | | 60/40 | 1.51 | 0.66 | 0.11 | 1.54 | 0.23 | 0.08 |
| | 40 | | | 75/25 | 1.52 | 0.53 | 0.10 | 1.55 | 0.13 | 0.07 |
| | 41 | | | 85/15 | 1.53 | 0.46 | 0.09 | 1.56 | 0.12 | 0.06 |
| | 42 | | D-2.6E | 100 | 1.54 | 0.24 | 0.08 | 1.57 | 0.12 | 0.05 |
| | 43 | | BisGMA | 100 | 1.55 | 0.16 | 0.07 | 1.57 | 0.10 | 0.05 |
| Comparative Example | 28 | RF7 (GdF$_3$) | D-2.6E/ 3G | 40/60 | 1.49 | 0.99 | 0.13 | 1.53 | 0.67 | 0.09 |
| | 29 | | | 50/50 | 1.50 | 0.98 | 0.12 | 1.54 | 0.62 | 0.08 |
| | 30 | | | 60/40 | 1.51 | 0.90 | 0.11 | 1.54 | 0.52 | 0.08 |
| | 31 | | | 75/25 | 1.52 | 0.81 | 0.10 | 1.55 | 0.48 | 0.07 |
| | 32 | | | 85/15 | 1.53 | 0.74 | 0.09 | 1.56 | 0.41 | 0.06 |
| | 33 | | D-2.6E | 100 | 1.54 | 0.64 | 0.08 | 1.57 | 0.34 | 0.05 |
| | 34 | | BisGMA | 100 | 1.55 | 0.60 | 0.07 | 1.57 | 0.31 | 0.05 |

As shown in Table 6, also in the GdF$_3$-based curable compositions, when Examples and Comparative Examples having the same composition of the polymerizable monomer(s) were compared to each other, it was recognized that both the curable composition and the cured body thereof had higher transparency (lower contrast ratio) in Examples.

5. Evaluation of X-ray Radiography Contrast

The curable compositions of Examples were each tested in conformity with ISO 13116-2014. The result was that the cured bodies obtained from the curable compositions of Examples each showed X-ray opacity higher than that of an aluminum material having the same thickness as the cured body. Accordingly, it was recognized that the cured bodies obtained from the curable compositions of Examples each had sufficient opacity to X rays. It is required that the cured body show, as practical X-ray opacity required for a dental material, X-ray opacity at around the same level as or a higher level than that of an aluminum material having the same thickness as the cured body. Herein, the blending amount of the X-ray opaque filler of the present invention, with which the cured body showed X-ray opacity at around the same level as that of an aluminum material having the same thickness as the cured body, was approximately from about 3 parts by mass to about 10 parts by mass with respect to 100 parts by mass of the curable composition.

The curable compositions of Examples described above can each be suitably used as a dental curable composition. In addition, for reference, various graphs created based on experimental data shown in Tables 1 to 6 are shown in FIG. 1 to FIG. 8.

**Claims**

1. An X-ray opaque filler, which is blended in a curable composition including a polymerizable monomer to impart X-ray opacity to the curable composition and a cured body thereof,
   the X-ray opaque filler comprising any powder selected from the group consisting of: first powder including crystalline rare earth metal fluoride particles as a main component and having a full width at half maximum of a maximum intensity peak derived from the crystalline rare earth metal fluoride particles of 0.3° or more in an X-ray diffraction pattern; and second powder obtained by subjecting the first powder to surface treatment.

2. The X-ray opaque filler according to claim 1, wherein the first powder is powder including, as a main component, at least one kind of particles selected from the group consisting of: crystalline rare earth metal fluoride particles having an average primary particle diameter of from 1 nm to 500 nm measured with an electron microscope; and aggregated particles of the crystalline rare earth metal fluoride particles having an average primary particle diameter of from 1 nm to 500 nm.

3. The X-ray opaque filler according to claim 1 or 2, wherein the crystalline rare earth metal fluoride particles are crystalline ytterbium fluoride particles.

4. The X-ray opaque filler according to claim 1 or 2, wherein the crystalline rare earth metal fluoride particles are at least one kind of particles selected from the group consisting of: crystalline lanthanum fluoride particles; crystalline cerium fluoride particles; and crystalline gadolinium fluoride particles.

5. The X-ray opaque filler according to any one of claims 1 to 4, wherein the full width at half maximum is 0.77° or less.

6. The X-ray opaque filler according to any one of claims 1 to 4, wherein the full width at half maximum is from 0.47° to 0.68°.

7. The X-ray opaque filler according to any one of claims 1 to 4, wherein the full width at half maximum is from 0.51° to 0.59°.

8. A dental X-ray opaque filler, comprising the X-ray opaque filler of any one of claims 1 to 7.

9. A method of producing the X-ray opaque filler of any one of claims 1 to 7, comprising a step of subjecting raw material powder including crystalline rare earth metal fluoride particles as a main component and having a full width at half maximum of a maximum peak derived from the crystalline rare earth metal fluoride particles of less than 0.3° in an X-ray diffraction pattern to mechanochemical treatment so that the full width at half maximum becomes 0.3° or more.

10. The method of producing the X-ray opaque filler according to claim 9, wherein the mechanochemical treatment is wet bead mill treatment.

11. A dental curable composition, comprising:

a polymerizable monomer; and
the X-ray opaque filler of any one of claims 1 to 8.

12. The dental curable composition according to claim 11,

wherein the crystalline rare earth metal fluoride particles are crystalline ytterbium fluoride particles, and
wherein a cured body of the polymerizable monomer has a refractive index for a sodium d line at 25°C of from 1.45 to 1.60.

13. The dental curable composition according to claim 11 or 12, wherein the dental curable composition satisfies the following expression (1):

$$\text{Expression (1)} \quad -0.02 \leq (n_X - n_M) \leq 0.1$$

in the expression (1), $n_X$ represents a refractive index of the crystalline rare earth metal fluoride particles, and $n_M$ represents a refractive index of a cured body of the polymerizable monomer.

## FIG. 1

## FIG. 2

**FIG. 3**

**FIG. 4**

YbF$_3$-40 BASED:F1-3h & RF1

Legend:
- ○ F1-3h_BEFORE CURING
- ● F1-3h_AFTER CURING
- ◇ RF1_BEFORE CURING
- ◆ RF1_AFTER CURING

X-axis: REFRACTIVE INDEX

Y-axis: CONTRAST RATIO (Yb/Yw)

**FIG. 5**

FIG. 6

EP 4 403 161 A1

CeF₃ BASED : F6-3h & RF6

FIG. 7

GdF$_3$ BASED : F7-3h & RF7

FIG. 8

EP 4 403 161 A1

<table>
<tr><td colspan="2">**INTERNATIONAL SEARCH REPORT**</td><td>International application No.<br>**PCT/JP2022/031237**</td></tr>
</table>

**A.    CLASSIFICATION OF SUBJECT MATTER**

*A61K 6/84*(2020.01)i; *A61K 6/16*(2020.01)i; *A61K 6/25*(2020.01)i
FI:    A61K6/84; A61K6/25; A61K6/16

According to International Patent Classification (IPC) or to both national classification and IPC

**B.    FIELDS SEARCHED**

Minimum documentation searched (classification system followed by classification symbols)

A61K6/84; A61K6/16; A61K6/25

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| A | 長谷部類 他, 歯科材料用途に向けたフッ化物フィラーの開発, セラミックス, 2019, 54(9), pages 646-649, (Ceramics.), non-official translation (HASEBE, Rui et al. Development of fluoride fillers for dental material applications.)<br>entire text | 1-13 |
| A | JP 3-17803 B2 (ETAB. DENTAIRE IVOCLAR) 11 March 1991 (1991-03-11)<br>entire text | 1-13 |
| A | JP 2008-266767 A (HITACHI CHEM. CO., LTD.) 06 November 2008 (2008-11-06)<br>entire text | 1-13 |
| A | JP 56-51407 A (COLGATE-PALMOLIVE CO.) 09 May 1981 (1981-05-09)<br>entire text | 1-13 |
| A | JP 2021-80196 A (KURARAY NORITAKE DENTAL INC.) 27 May 2021 (2021-05-27)<br>entire text | 1-13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 October 2022** | **25 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/031237**

| Patent document cited in search report | | | Publication date (day/month/year) | Patent family member(s) | | | Publication date (day/month/year) |
|---|---|---|---|---|---|---|---|
| JP | 3-17803 | B2 | 11 March 1991 | JP | 61-176508 | A | |
| | | | | US | 4629746 | A | |
| | | | | EP | 189540 | A2 | |
| JP | 2008-266767 | A | 06 November 2008 | JP | 2012-136778 | A | |
| | | | | US | 2008/0241368 | A1 | |
| | | | | US | 2012/0111232 | A1 | |
| | | | | CN | 101276666 | A | |
| JP | 56-51407 | A | 09 May 1981 | (Family: none) | | | |
| JP | 2021-80196 | A | 27 May 2021 | (Family: none) | | | |

Form PCT/ISA/210 (patent family annex) (January 2015)

**EP 4 403 161 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3017803 B